(19) Europäisches Patentamt
European Patent Office
Office européen des brevets

(11) Veröffentlichungsnummer: **0 298 326 B1**

# (12) EUROPÄISCHE PATENTSCHRIFT

(45) Veröffentlichungstag der Patentschrift: **16.12.92**

(51) Int. Cl.[5]: **C07D 233/16**

(21) Anmeldenummer: **88110157.0**

(22) Anmeldetag: **25.06.88**

Die Akte enthält technische Angaben, die nach dem Eingang der Anmeldung eingereicht wurden und die nicht in dieser Patentschrift enthalten sind.

## (54) Verfahren zur Herstellung von in 2-Stellung substituierten 1-(Acylaminoalkyl)-2-imidazolinen.

(30) Priorität: **04.07.87 DE 3722186**

(43) Veröffentlichungstag der Anmeldung:
**11.01.89 Patentblatt 89/02**

(45) Bekanntmachung des Hinweises auf die Patenterteilung:
**16.12.92 Patentblatt 92/51**

(84) Benannte Vertragsstaaten:
**CH DE ES FR GB IT LI**

(56) Entgegenhaltungen:
**EP-A- 0 186 967**
**DE-A- 3 620 218**
**FR-B- 1 582 293**

(73) Patentinhaber: **CASSELLA Aktiengesellschaft**
**Hanauer Landstrasse 526**
**W-6000 Frankfurt am Main 61(DE)**

(72) Erfinder: **Keil, Karl-Heinz, Dr.**
**Lübecker Weg 3**
**W-6450 Hanau-Mittelbuchen(DE)**
Erfinder: **Eckardt, Georg-Wolfgang, Dr.**
**Hünfelder Strasse 18**
**W-6000 Frankfurt am Main 61(DE)**
Erfinder: **Wirtz, Herbert, Dr.**
**Rosserstrasse 31**
**W-6239 Eppstein/Ts.(DE)**
Erfinder: **Berenbold, Helmut, Dr.**
**Eberlein-Strasse 25**
**W-6200 Wiesbaden(DE)**
Erfinder: **Wellbrock, Werner, Dr.**
**Dreilindenstrasse 30**
**W-6232 Bad Soden/Ts.(DE)**
Erfinder: **Fröhlich, Horst, Dr.**
**Eichenweg 19**
**W-6239 Eppstein/Ts.(DE)**

(74) Vertreter: **Urbach, Hans-Georg, Dr. et al**
**Hanauer Landstrasse 526**
**W-6000 Frankfurt am Main 61(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von in 2-Stellung substituierten 1-(Acylaminoalkyl)-2-imidazolinen der allgemeinen Formel I

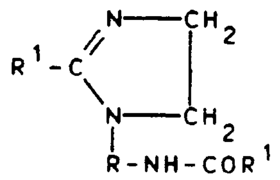

(I)

worin
R einen Alkylenrest der Formel $-CH_2CH_2-$, $-CH_2CH_2-CH_2-$ oder $-CH(CH_3)CH_2CH_2-$ und
$R^1$ einen Fettsäurerest mit 7 bis 25 C-Atomen
bedeuten.

Verbindungen der Formel I sind für R = Ethylen und Trimethylen bekannt und werden insbesondere zur Herstellung der entsprechenden quarternierten Imidazoliniumsalze benötigt (vgl. Kirk-Othmer, Encyclopedia of Chemical Technology, Third Edition, Volume 19, (1982), Seite 527), die ihrerseits wichtige technische Tenside darstellen. Diese kommen infolge ihres milden und hautfreundlichen Charakters besonders im Kosmetik- und Körperpflegebereich zur Anwendung. Verbindungen der Formel I mit R = Ethylen oder Trimethylen werden auch als Komponenten bei der Formulierung von Waschmitteln und Korrosionsschutzmitteln in beträchtlichen Mengen eingesetzt.

Zur Herstellung der Verbindungen der Formel I mit R = Ethylen und Trimethylen ist es bekannt, eine Fettsäure der allgemeinen Formel II

$R^1COOH$ (II)

worin $R^1$ die bereits angegebene Bedeutung besitzt, mit einem Dialkylentriamin der Formel III

$H_2N-CH_2-CH_2-NH-R-NH_2$ (III)

worin R = Ethylen ($-CH_2CH_2-$) oder Trimethylen ($-CH_2CH_2CH_2-$) bedeutet, umzusetzen. Für eine vollständige Umsetzung ist stöchiometrisch ein Molverhältnis Fettsäure : Dialkylentriamin = 2,0 : 1 erforderlich.

Zur Herstellung einer Verbindung der Formel I wird beispielsweise nach dem typischen Beispiel 2 der französischen Patentschrift 1 582 293 eine Mischung von Behensäure und Diethylentriamin im Molverhältnis 2 : 1 in Xylol 15 h lang in einer Stickstoffatmosphäre unter vermindertem Druck auf 90 bis 100 °C erhitzt und dabei das entstehende Reaktionswasser azeotrop abdestilliert. Anschließend wird das Xylol unter vermindertem Druck abdestilliert. Das Arbeiten in Xylol und der Zwang zur Rückgewinnung des Xylols ist umständlich und verteuert das Verfahren.

Aus der europäischen Patentanmeldung 186 967 ist es bei der Herstellung von Fettsäureamidopropylimidazolinen bekannt, etwa 2 mol Aminopropyl-ethylendiamin mit etwa 2 mol Fettsaure oder einem amidbildenden Derivat einer Fettsäure zu einem Fettsäurediamid umzusetzen und dieses dann zum Imidazolin unter azeotroper Wasserabspaltung zu cyclisieren. Die Reaktion und die Cyclisierung werden in Toluol bei Temperaturen von 70 bis 150 °C durchgeführt. Das Arbeiten in Toluol ist umständlich und verteuert das Verfahren.

Es ist auch hereits bekannt, zur Herstellung von Verbindungen der Formel I mit R = Ethylen und Trimethylen die Fettsäure der Formel II und das Dialkylentriamin der Formel III mit R = Ethylen und Trimethylen ohne Anwendung eines Lösungsmittels umzusetzen. Die Schwierigkeiten bei der Herstellung der Verbindungen der Formel I durch Umsetzung von Fettsäuren der Formel II mit Dialkylentriamin der Formel III bestehen unter anderem darin, daß die Reaktion in zwei Schritten verläuft, wobei zunächst das Dialkylentriamin durch die Fettsäure acyliert und dieses Zwischenprodukt dann unter Ringschluß in die Verbindung der Formel I übergeführt wird und für ein technisch brauchbares Verfahren zur Herstellung der Verbindungen der Formel I gefordert wird, daß die Ausbeute an den Verbindungen der Formel I hoch ist und in dem erhaltenen Produkt nur vernachlässigbare Mengen an den Ausgangsprodukten sowie möglicher Acylierungszwischenprodukte der Formeln IV bis X

$$R^1CO-NH-CH_2-CH_2-NH-R-NH_2 \qquad (IV)$$

$$H_2N-CH_2-CH_2-NH-R-NH-COR^1 \qquad (V)$$

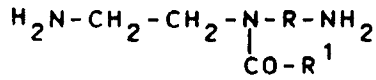

(VI)

$$R^1CO-HN-CH_2-CH_2-NH-R-NH-COR^1 \qquad (VII)$$

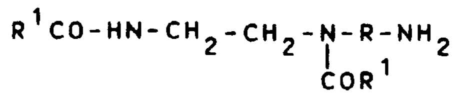

(VIII)

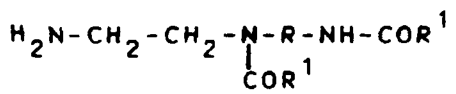

(IX)

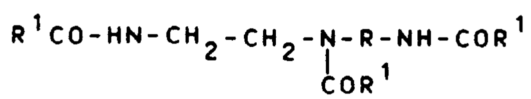

(X)

und anderer Imidazolinverbindungen, beispielsweise solcher der allgemeinen Formel XI

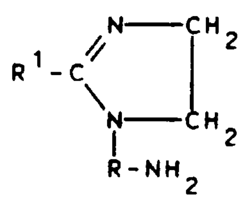

(XI)

vorhanden sind. Für R = Ethylen sind die vorstehend angegebenen Formeln IV und V sowie VIII und IX untereinander identisch.

Aus den Untersuchungen von Raymond G. Bistline, James W. Hampson and Warner M Linfield in JAOCS, Vol.60, No.4, (April 1983), Seiten 823 bis 828, insbesondere Seite 826, ist es bekannt, daß bei der Umsetzung einer Fettsäure der Formel II mit Diethylentriamin bei einer Temperatur von 150 °C überwiegend ein Zwischenprodukt der vorgenannten Formel VIII gebildet wird. Dieses Zwischenprodukt kann dann anschließend durch Erhitzen bei 150 °C unter einem verminderten Druck von 0,2 mm/Hg in das gewünschte Imidazolin der Formel I überführt werden. Bei einer Cyclisierungsdauer von 6 Stunden werden bei diesem Verfahren bei Laboransätzen Ausbeuten von 95 % an dem gewünschten Imidazolin der allgemeinen Formel I erhalten. Bei einer Ansatzvergrößerung treten durch die erheblichen Wassermengen, die währen der Reaktion aus dem Reaktionsgemisch abzuführen sind, beträchtliche verfahrenstechnische Schwierigkeiten auf, die letztendlich bewirken, daß sich die guten, bei Laboransätzen erzielbaren Ausbeuten und Reinheiten bei technischen Ansätzen nicht verwirklichen lassen.

In dem Verfahren der DE-A1-36 20 218 wird die Umsetzung zwischen der Fettsäure der Formel II, wobei $R^1$ eine Alkyl- oder Alkenylgruppe mit 8 bis 22 C-Atomen bedeutet, und einem Dialkylentriamin der Formel III mit R = Ethylen und Trimethylen so gesteuert, daß dabei zunächst ein Produkt entsteht, das überwiegend aus einer Imidazolinverbindung der vorgenannten Formel XI besteht. Diese Imidazolinverbindung der Formel XI wird dann anschließend durch weitere Umsetzung mit der Fettsäure der Formel II in die gewünschte Verbindung der allgemeinen Formel I übergeführt.

Bei dem Verfahren der DE-A1-36 20 218 zur Herstellung von Verbindungen der Formel I mit R =

Ethylen oder Trimethylen wird

1) in einen Reaktor Dialkylentriamin mit R = Ethylen und Trimethylen zusammen mit einer höheren Fettsäure der Formel II, wobei $R^1$ eine Alkyl- oder Alkenylgruppe mit 8 bis 22 C-Atomen bedeutet, oder einem Ester davon in einem Molverhältnis von Fettsäure oder Fettsäureester: Dialkylentriamin von 1,5 : 1 bis 1,8 : 1 eingeführt und

2) die Reaktion bei einer Innentemperatur von 100 bis 250 °C unter vermindertem Druck durchgeführt und

3) der Reaktionsmischung die höhere Fettsäure oder ein Ester davon zugesetzt, so daß ein Molverhältnis Fettsäure oder Fettsäureester und Dialkylentriamin von mindestens 2,0 : 1 erreicht werden kann und

4) die Reaktion bei einer Innentemperatur von 100 bis 250 °C unter vermindertem Druck fortgesetzt.

Nach dem typischen Beispiel 1 der DE-A1-36 20 218 wurden' Rindertalgfettsäure und Diethylentriamin im Molverhältnis 1,8 3 h lang auf 150 °C bei einem Druck von 400 mmHg erhitzt. Dann wurde die Temperatur 4 h lang auf 230 °C erhöht und der Druck gleichzeitig auf 30 mmHg erniedrigt. Dann wurde das Reaktionsgemisch abgekühlt und weitere Rindertalgfettsäure zugesetzt. Anschließend wurde 8 h lang auf 230 °C unter einem Druck von 30 mmHg erhitzt. Die Reaktionszeit beträgt somit insgesamt 15 h, und der Zwang, sämtliche Stufen unter vermindertem Druck durchzuführen, verteuert das Verfahren.

Außerdem treten bei diesem Verfahren bei der Übertragung in den großtechnischen Maßstab insbesondere zwei wesentliche Nachteile auf:

1. Durch das Arbeiten unter Unterdruck werden nicht kontrollierbare Anteile von Dialkylentriamin zusammen mit dem bei der Reaktion entstehenden Wasser abdestilliert, so daß bei technischen Verfahren das gewünschte Molverhältnis nicht genau eingehalten werden kann.

2. Durch die gemeinsame Umsetzung von Fettsäure und Dialkylentriamin bei Temperaturen von 140 bis 170 °C werden beträchtliche Mengen an Trisamiden der vorstehend genannten Formel X gebildet. Diese sind nicht erwünscht und tragen zur Minderung der Wirksamkeit der Verbindungen mit der allgemeinen Formel I bei.

Überraschenderweise wurde nun gefunden, daß es möglich ist, die Nachteile der bisherigen Verfahren zur Herstellung der Verbindungen der Formel I zu vermeiden. Die Erfindung betrifft ein Verfahren zur Herstellung von Verbindungen der allgemeinen Formel I durch Umsetzung einer Fettsäurekomponente, bestehend aus einer oder mehreren Fettsäuren der allgemeinen Formel II und/oder einem oder mehreren Estern von Fettsäuren der Formel II und/oder einem oder mehreren Glyzeriden von Fettsäuren der Formel II mit einem Dialkylentriamin der Formel III und ist dadurch gekennzeichnet, daß die Fettsäurekomponente und das Di-alkylentriamin der Formel III im Molverhältnis (1,9 bis 2,0) : 1 derart umgesetzt wird, daß das Dialkylentriamin unter Inertgasatmosphäre vorgelegt und auf eine Temperatur von 100 bis 190 °C gebracht und die Fettsäurekomponente flüssig mit einer Temperatur von 100 bis 190 °C zudosiert wird und das bei der Reaktion entstehende Wasser und/oder Glyzerin und/oder der bei der Reaktion entstehende Alkohol abdestilliert und die Amidoaminbildung durch Erhitzen auf Temperaturen von 140 bis 190 °C vervollständigt und danach bei einem Unterdruck von mindestens 50 mbar die Cyclisierung zu der Verbindung der Formel I durchgeführt wird.

In den Verbindungen der Formel I steht der Fettsäurerest $R^1$ mit 7 bis 25 C-Atomen insbesondere für eine Alkyl-oder Alkenylgruppe. Die Alkyl- und Alkenylgruppe kann geradkettig oder verzweigt und kann auch mit OH- substituiert sein. Die Alkenylgruppe kann ein- oder mehrfach, beispielsweise zwei-, drei- oder vierfach ungesättigt sein. Der für $R^1$ stehende Fettsäurerest leitet sich von Fettsäuren mit 8 bis 26 C-Atomen durch Abspaltung der Carboxylgruppe ab. Dementsprechend kann die als Ausgangsprodukt einzusetzende Fettsäurekomponente z.B. eine oder mehrere Fettsäuren der allgemeinen Formel II mit 8 bis 26 C-Atomen enthalten. Beispiele für derartige Fettsäuren sind: Caprylsäure, Pelargonsäure, Caprinsäure, Undecansäure, Laurinsäure, Myristinsäure, Palmitinsäure, Margarinsäure, Stearinsäure, Arachinsäure, Behensäure, Lignocerinsäure, Cerotinsäure, Tuberculostearinsäure, Stilingasäure, Palmitoleinsäure, Ölsäure, Ricinoleinsäure, Petroselinsäure, Vaccensäure, Linolsäure, Linolensäure, Eläostearinsäure, Licansäure, Parinarsäure, Arachidonsäure, Erucasäure, Selacholeinsäure. Auch Mischungen derartiger Fettsäuren können eingesetzt werden, insbesondere Mischungen von Fettsäuren, wie sie aus festen, halbfesten oder flüssigen Fetten, beispielsweise Kokosnußöl, Palmkernöl, Olivenöl, Ricinusöl, Rapsöl, Erdnußöl, Palmfett, Schweineschmalz oder Rindertalg, gewonnen werden können.

Die als Ausgangsprodukt einzusetzende Fettsäurekomponente kann die Fettsäure der Formel II auch in Form eines Esters, insbesondere eines Alkylesters mit 1 bis 4 C-Atomen, vorzugsweise eines Methyl- oder Ethylesters, und/oder in Form eines Glyzerids enthalten, so daß auch Fette und Öle, insbesondere solche, welche die vorgenannten Fettsäuren enthalten, eingesetzt werden können. Die Fettsäurekomponente kann aus einer Fettsäure, einem Ester oder einem Glyzerid oder aus einer Mischung derartiger Verbindungen bestehen. So können z.B. die Ester und/oder Glyzeride (d.h. Fette oder Öle) auch in Mischungen

untereinander und/oder in Mischungen mit den Fettsäuren eingesetzt werden. Mischungen von Fettsäuren mit Glyzeriden, d.h. Fettsäuren mit Fetten und/oder Ölen, sind bevorzugt.

Als Dialkylentriamin der allgemeinen Formel III kann das Diethylentriamin der Formel XII

$H_2N\text{-}CH_2\text{-}CH_2\text{-}NH\text{-}CH_2\text{-}CH_2\text{-}NH_2$ (XII)

oder das Triamin der Formel XIII

$H_2N\text{-}CH_2\text{-}CH_2\text{-}NH\text{-}CH_2\text{-}CH_2\text{-}CH_2\text{-}NH_2$ (XIII)

oder das Triamin der Formel XIV

$$H_2N\text{-}CH_2\text{-}CH_2\text{-}NH\text{-}\underset{\displaystyle CH_3}{\underset{|}{CH}}\text{-}CH_2\text{-}CH_2\text{-}NH_2 \qquad (XIV)$$

eingesetzt werden. Die Verbindungen der Formeln XII und XIII sind bekannt. Das Triamin der Formel XIV kann technisch durch Anlagerung von Ethylendiamin an Crotonsäurenitril und anschließende Hydrierung hergestellt werden.

Es ist auch möglich, Mischungen dieser drei Triamine zu verwenden. Für R ist der Ethylenrest ($-CH_2CH_2$)- bevorzugt, was bedeutet, daß als Ausgangsprodukt der allgemeinen Formel III vorzugsweise das Diethylentriamin der Formel XII eingesetzt wird.

Bei der Durchführung des erfindungsgemäßen Verfahrens wird das Dialkylentriamin der Formel III unter Inertgasatmosphäre vorgelegt. Als Inertgas ist jedes Gas geeignet, das unter den Reaktionsbedingungen nicht mit den Ausgangs- und Endproduckten reagiert. Geeignet sind beispielsweise Edelgase, insbesondere aber Stickstoff, der wegen seiner Preisgünstigkeit bevorzugt wird.

Nachdem das Dialkylentriamin unter Inertgasatmosphäre vorgelegt worden ist, wird es auf eine Temperatur von 100 bis 190 °C erhitzt, und dem erhitzten und zweckmäßigerweise gerührten Dialkylentriamin wird dann die auf eine Temperatur von 100 bis 190 °C erhitzte Fettsäurekomponente zudosiert, wobei die Amidoaminbildung sofort unter Abspaltung von Wasser, Alkohol und/oder Glyzerin einsetzt. Zweckmäßigerweise wird die Fettsäurekomponente mit der Temperatur zudosiert, die das vorgelegte Dialkylentriamin bzw. das Reaktionsgemisch besitzt.

Wenn die Fettsäurekomponente aus einen oder mehreren Ester von Fettsäuren der Formel II besteht, wird das Dialkylentriamin vorzugsweise auf eine Temperatur von 100 bis 150 °C, ganz besonders bevorzugt auf 110 bis 140 °C, erhitzt und die Fettsäurekomponente vorzugsweise mit einer Temperatur von 100 bis 150 °C, ganz besonders bevorzugt 110 bis 130 °C, zudosiert.

Wenn die Fettsäurekomponente aus einer oder mehreren Fettsäuren der Formel II besteht, wird das Dialkylentriamin vorzugsweise auf eine Temperatur von 130 bis 190 °C, ganz besonders bevorzugt auf 150 bis 170 °C, erhitzt und die Fettsäurekomponente vorzugsweise mit einer Temperatur von 130 bis 170 °C, ganz besonders bevorzugt 140 bis 160 °C, zudosiert.

Wenn die Fettsäurekomponente aus einem oder mehreren Glyzeriden von Fettsäuren der Formel II besteht, kommen für die Erhitzung des Dialkylentriamins und der Zudosierung der Fettsäurekomponente die gleichen bevorzugten und ganz besonders bevorzugten Temperaturbereiche in Betracht, die vorstehend für eine aus Fettsäuren bestehende Fettsäurekomponente angegeben sind.

Der bei der Reaktion gebildete Alkohol bzw. das gebildete Wasser wird vorteilhafterweise sofort laufend abdestilliert, was zweckmäßigerweise durch einen aufgesetzten absteigenden Kühler erfolgt. Es ist zweckmäßig, die Geschwindigkeit für die Zudosierung der heißen flüssigen Fettsäure der allgemeinen Formel II so zu wählen, daß im Kopf des absteigenden Kühlers das Destillat eine Temperatur von 90 bis 100 °C besitzt. Durch diese Kopftemperatur des übergehenden Destillats kann die Dosiergeschwindigkeit auch automatisch gesteuert werden. Auch bei Verwendung eines Esters kann die Dosiergeschwindigkeit durch die Temperatur des Destillats im absteigenden Kühler automatisch gesteuert werden. Bei der Durchführung des Verfahrens im technischen Maßstab kann in der Regel die heiße flüssige Fettsäurekomponente mit einer Geschwindigkeit von 150 bis 800 l/h, vorzugsweise 250 bis 600 l/h, zudosiert werden.

Wenn die Fettsäurekomponente aus einem Glyzerid besteht oder ein Glyzerid enthält, wird bei der Reaktion Glyzerin abgespalten. Dieses Glyzerin wird nach der Zudosierung der Fettsäurekomponente während der Amidoaminbildung und/oder Cyclisierung unter Unterdruck von mindestens 50 mbar, zweckmäßigerweise von mindestens 2 mbar, abdestilliert.

Wenn die Fettsäurekomponente aus einer oder mehreren Fettsäuren der Formel II und/oder einem oder mehreren Glyzeriden besteht, dann wird das vorgelegte Dialkylentriamin der Formel III bzw. das entstandene Reaktionsgemisch während der Zudosierung vorzugsweise auf einer Temperatur von 130 bis 190 °C, ganz besonders bevorzugt von 150 bis 170 °C, gehalten. Wenn die Fettsäurekomponente aus einem oder mehreren Estern von Fettsäuren der Formel II besteht, dann wird das vorgelegte Dialkylentriamin der Formel III bzw. das entstandene Reaktionsgemisch während der Zudosierung vorzugsweise auf einer Temperatur von 100 bis 150 °C, ganz besonders bevorzugt von 110 bis 140 °C, gehalten.

Bei der Verwendung von Mischungen als Fettsäurekomponente kann die Mischung dem vorgelegten Dialkylentriamin zugesetzt werden, oder es werden die Einzelbestandteile der Mischung und/oder die Mischung in beliebiger Reihenfolge dem vorgelegten Dialkylentriamin bzw. dem Reaktionsansatz zugefügt.

Bei der Verwendung eines Alkylesters einer Fettsäure der Formel II ist es zur Reaktionsbeschleunigung bevorzugt, bezogen auf Ester dem Reaktionsgemisch etwa 0,01 bis 2,0 Gew.%, in der Regel ca. 0,03 bis 0,5 Gew.%, einer starken Base, wie z.B. eines Alkalialkoholats, wie z.B. Natrium-oder Kalium-methylat oder -ethylat, oder eines Alkalihydroxids, wie z.B. Natrium- oder Kaliumhydroxid, zuzusetzen.

Die Menge der bei dem erfindungsgemäßen Verfahren dem vorgelegten Dialkylentriamin der Formel III zudosierten Fettsäurekomponente wird so gewählt, daß das Molverhältnis zwischen Fettsäure der Formel II und Dialkylentriamin der Formel III (1,9 bis 2,0) : 1 beträgt. Bei der Berechnung des Molverhältnisses ist bei der Verwendung von Glyzeriden zu berücksichtigen, daß diese im Molekül nicht nur einen Fettsäurerest, sondern im Fall der natürlichen Öle und Fette drei Fettsäurereste enthalten. 1 Mol Triglyzerid geht also mit 3 Molen Fettsäureresten in die Berechnung des Molverhältnisses ein.

Nach der Zudosierung der Fettsäurekomponente wird zur Vervollständigung der Amidoaminbildung weiter auf eine Temperatur von 140 bis 190 °C, vorzugsweise 150 bis 170 °C, erhitzt. Die Amidoaminbildung ist abgeschlossen, wenn bei der Verwendung eines Esters kein Alkohol und bei der Verwendung einer Fettsäure kein Wasser mehr entweicht. Es ist anzunehmen, daß sich bei dem erfindungsgemäßen Verfahren dabei überwiegend das Diamidoamin der eingangs erwähnten Formel VIII bildet. Normalerweise kann die sich nach der Beendigung der Zudosierung der Fettsäure der Formel II oder ihres Esters anschließende Kondensationsphase zur Vervollständigung der Amidoaminbildung nach 2 bis 4 Stunden, insbesondere nach 2 1/2 bis 3 Stunden, beendet werden.

Die anschließende Cyclisierung wird bei einem Unterdruck von mindestens 50 mbar, beispielsweise bei einem Unterdruck von 50 bis 0,01 mbar, vorzugsweise bei 50 bis 0,1 mbar, durchgeführt. Bei der Cyclisierung wird normalerweise eine Temperatur von mindestens 140 °C, insbesondere mindestens 170 °C, vorzugsweise mindestens 180 °C, eingehalten. Die Cyclisierung kann z.B. im Temperaturbereich von 140 bis 230 °C, insbesondere 170 bis 210 °C und vorzugsweise im Temperaturbereich von 180 bis 190 °C, durchgeführt werden. Die Cyclisierung ist in den meisten Fällen nach 4 bis 6 Stunden beendet.

Falls die Fettsäurekomponente ein Glyzerid enthält, wird das bei der Reaktion mit dem Dialkylentriamin der Formel III abgespaltene Glyzerin ganz oder zum Teil während der Kondensationsphase durch Anlegung eines Unterdrucks, insbesondere eines Unterdrucks von mindestens 50 mbar, z.B. bei einem Unterdruck von 0,01 bis 50 mbar, vorzugsweise 50 bis 0,1 mbar, abdestilliert. Die Kondensationsphase kann dabei gleitend in die Phase der Cyclisierung übergehen.

Zur Erzielung hellfarbiger bzw. farbloser Verbindungen der allgemeinen Formel I ist es zweckmäßig, das erfindungsgemäße Verfahren in Anwesenheit von 0,2 bis 5 Gew.% phosphoriger und/oder unterphosphoriger Säure, bezogen auf das Gesamtgewicht der Ausgangsverbindungen der Formel II und III durchzuführen. Die phosphorige und/oder unterphosphorige Säure kann dabei, gegebenenfalls in Form von wäßrigen Lösungen, sowohl dem vorgelegten Dialkylentriamin der allgemeinen Formel III, als auch der Fettsäure der allgemeinen Formel II oder ihren Ester zugegeben werden. Es ist auch möglich, die zur Anwendung kommende Menge der phosphorigen und/oder unterphosphorigen Säure auf beide Ausgangsprodukte der Formel II und III aufzuteilen. Die Verwendung von unterphosphoriger Säure ist bevorzugt.

Das erfindungsgemäße Verfahren wird ohne Anwendung eines Lösungsmittels durchgeführt und läuft bis zu der abschließenden Cyclisierung ohne Anwendung eines Über- oder Unterdrucks ab. Bei dem erfindungsgemäßen Verfahren treten durch das Entweichen des bei der Reaktion gebildeten wassers oder Alkohols kein Schäumen oder Spritzen oder andere Schwierigkeiten auf. Die Gesamtumsetzungszeiten sind mit durchschnittlich 7 bis 9 Stunden relativ gering und ermöglichen dadurch Raum-Zeit-Ausbeuten, die den bisherigen Verfahren überlegen sind. Die Ausbeuten an den Verbindungen der allgemeinen Formel I sind hoch und liegen in der Regel bei 90 bis 98 % der Theorie. Die nach dem erfindungsgemäßen Verfahren herstellbaren Verbindungen der Formel I sind so rein, daß sie ohne weitere Reinigungs- oder Nachbehandlungsoperationen direkt für die verschiedensten Zwecke weiterverwendet werden können. Insbesondere weisen die nach dem erfindungsgemäßen Verfahren herstellbaren Imidazonlinverbindungen der Formel I

einen vernachlässigbaren Gehalt der besonders unerwünschten Trisamidoverbindungen der allgemeinen Formel X auf.

Die Verbindungen der Formel I mit R = Methyl-trimethylen (= $-CH(CH_3)CH_2CH_2-$) sind neu und werden im Rahmen der vorliegenden Erfindung beansprucht. Sie besitzen Eigenschaften, die denen der bekannten Verbindungen der Formel I mit R = Ethylen ($-CH_2CH_2-$) und Trimethylen ($-CH_2CH_2CH_2-$) ähnlich sind und können für die gleichen Zwecke verwendet werden, wie die Verbindungen der Formel I mit R = Ethylen oder Trimethylen ($-CH_2CH_2-$ oder $-CH_2CH_2CH_2-$), also insbesondere als Ausgangsprodukte für die Herstellung entsprechender quaternärer Stickstoffverbindungen mit oberflächenaktiven Eigenschaften. Sie können aber auch vorzugsweise in Kombination mit anderen quaternären Verbindungen für die Herstellung von Formulierungen für die Behandlung von Textilien eingesetzt werden.

Das erfindungsgemäße Verfahren wird durch die nachfolgenden Beispiele 1 bis 16 weiter erläutert. Die nachfolgenden Beispiele 9 und 10 stellen Vergleichsbeispiele dar, die zeigen, daß man bei nichterfindungsgemäßer Arbeitsweise nur wesentlich geringere Ausbeuten und unreinere Produkte erhält. Angegebene Prozente stellen, sofern nichts anderes angegeben ist, Gewichtsprozente dar.

Beispiel 1

In einem 3-l-Vierhalskolben mit einem Ankerrührer, heizbarem Tropftrichter, Thermometer und absteigendem Kühler werden nach dreimaligem Evakuieren und Spülen mit Stickstoff 148,4 g (1,438 mol) Diethylentriamin und 0,72 g unterphosphoriger Säure (in Form einer 50 gew.%igen wäßrigen Lösung) vorgelegt, mit Stickstoff überlagert und unter Rühren auf 155 bis 160 °C Innentemperatur geheizt. Dann werden 757,2 g (2,74 mol) Talgfettsäure aus dem beheizten Tropftrichter mit einer Temperatur von 135 °C in 60 s zugegeben; das entstehende Kondensationswasser wird dabei laufend über Kopf am absteigenden Kühler abdestilliert. Nach der Zugabe der Stearinsäure wird 3 h bei 150 bis 170 °C kondensiert, anschließend 6 h bei 185 bis 190 °C/4 mbar der Ringschluß zur Imidazolinverbindung durchgeführt.

Ansatzmolverhältnis: Talgfettsäure : Diethylentriamin = 1,905 : 1.

Ausbeute: 95,7 % d.Th. Imidazolinverbindung der Formel I mit R = Ethylen ($-CH_2CH_2-$) und $R^1$ einem Rest, der sich von der Talgfettsäure durch Abspaltung der Carboxylgruppe ableitet.

| Analyse: | gefunden | berechnet |
|---|---|---|
| N (gesamt nach Kjeldahl) | 7,0 % | 7,01 % |
| N (basisch, gesamt) | 2,35 % | 2,33 % |
| N (tertiär) | 2,21 % | 2,33 % |
| N (primär) | 0,09 % | 0 % |
| N (sekundär) | ≪0,01 % | 0 % |

Säurezahl: 2,3 (entsprechend 1,1 % freier Talgfettsäure)

Beispiel 2

Bei einer Wiederholung des Beispiels 1 werden anstelle der Talgfettsäure 750,8 g (2,74 mol) technische Stearinsäure mit einer Säurezahl von 205 eingesetzt. Die übrigen Bedingungen bleiben unverändert.

Ausbeute: 94,4 % d.Th. 1-(2-Stearoylamino-ethyl)-2-heptadecyl-2-imidazolin

| Analyse: | gefunden | berechnet |
|---|---|---|
| N (gesamt nach Kjeldahl) | 6,95 % | 7,01 % |
| N (basisch, gesamt) | 2,21 % | 2,33 % |
| N (tertiär) | 2,20 % | 2,33 % |
| N (primär) | 0,01 % | 0 % |
| N (sekundär) | ≪0,01 % | 0 % |

Beispiel 3

In einem 3-l-Vierhalskolben mit einem Ankerrührer, heizbarem Tropftrichter, Thermometer und absteigendem Kühler werden nach dreimaligem Evakuieren und Spülen mit Stickstoff 148,4 g (1,438 mol) Diethylentriamin und 0,72 g unterphosphoriger Säure (in Form einer 50 gew.%igen wäßrigen Lösung) vorgelegt, mit Stickstoff überlagert und unter Rühren auf 155 bis 160 °C Innentemperatur geheizt. Dann werden 804,2 g eines technischen Arachin-/Behensäure-Gemisches mit einer Säurezahl von 191 (2,74 mol) zusammen mit 0,723 g unterphosphoriger Säure (in Form einer 50 gew.%igen wäßrigen Lösung) aus einem beheizten Tropftrichter mit einer Temperatur von 155 bis 160 °C in 60 min zugegeben; das entstehende Kondensationswasser wird dabei laufend über Kopf am absteigenden Kühler abdestilliert. Nach der Zugabe des Arachin-/Behensäure-Gemisches wird 3 h bei 150 bis 170 °C kondensiert und anschließend 4 h bei 180 bis 190 °C und einem Vakuum von 40 mbar erhitzt.

Ansatzmolverhältnis: Arachin-/Behensäure : Diethylentriamin = 1,9505 : 1.

Ausbeute: 97,4 % Imidazolin-Verbindung der Formel I mit R = Ethylen ($-CH_2CH_2-$) und $R^1$ einem Rest, der sich von der Arachin-/Behensäure durch Abspaltung der Carboxylgruppe ableitet.

| Analyse: | gefunden | berechnet |
|---|---|---|
| N (gesamt nach Kjeldahl) | 6,60 % | 6,60 % |
| N (basisch, gesamt) | 2,05 % | 2,03 % |
| N (tertiär) | 1,97 % | 1,98 % |

Säurezahl: 2,4 (entsprechend 1,2 % freies Arachin-/Behensäure-Gemisch)

Beispiel 4

In einem 3-l-Vierhalskolben, der mit einem Ankerrührer, heizbarem Tropftrichter, Thermometer und absteigendem Kühler versehen ist, werden nach dreimaligem Evakuieren und Spülen mit Stickstoff 142,5 g (1,38 mol) Diethylentriamin vorgelegt mit Stickstoff überlagert und unter Rühren auf eine Innentemperatur von 155 bis 160 °C hochgeheizt und 756,2 g (2,74 mol) technische Stearinsäure mit einer Säurezahl von 203 und einer Temperatur von 140 °C flüssig dem Reaktionskolben zudosiert und das entstehende Kondensationswasser über Kopf am absteigenden Kühler abdestilliert..

Dann wird nach der Zugabe 3 h bei 160 bis 175 °C geheizt und anschließend in 5 h unter einem Vakuum von 10 mbar und einer Temperatur von 185 bis 190 °C zum Imidazolin umgesetzt.

Ansatzmolverhältnis: technische Stearinsäure : Diethylentriamin = 1,985 : 1.

Ausbeute:

92,5 % d.Th. Imidazolinverbindung der Formel I mit R = Ethylen ($-CH_2CH_2-$) und $R^1$ einem Rest, der sich von der technischen Stearinsäure durch Abspaltung der Carboxylgruppe ableitet.

| Analyse: | gefunden | berechnet |
|---|---|---|
| N (gesamt nach Kjeldahl) | 6,95 % | 7,01 % |
| N (basisch, gesamt) | 2,35 % | 2,33 % |
| N (tertiär) | 2,15 % | 2,33 % |

Säurezahl: 4,5 (entsprechend 2,2 % freier technischer Stearinsäure)

Beispiel 5

In einem 3-l-Vierhalskolben, der mit einem Ankerrührer, heizbarem Tropftrichter, Thermometer und absteigendem Kühler versehen ist, werden nach dreimaligem Evakuieren und Spülen mit Stickstoff 144,85 g (1,406 mol) Diethylentriamin und 0,71 g unterphosphoriger Säure (in Form einer 50 gew.%igen wäßrigen Lösung) vorgelegt, mit Stickstoff gespült und auf eine Innentemperatur von 170 °C geheizt. Dann werden aus einem heizbaren Tropftrichter 756,2 g (2,74 mol) Talgfettsäure flüssig mit einer Temperatur von 150 °C zugegeben. Das entstehende Wasser bei der Reaktion wird über Kopf an einer Kolonne am absteigenden Kühler abdestilliert. Anschließend wird 3 h bei 160 bis 170 °C kondensiert, wobei noch laufend Wasser abdestilliert. Danach wird durch Erhitzen auf 185 bis 190 °C unter einem Vakuum von 50 mbar in 4 h das gewünschte Imidazolin erhalten.

Ansatzmolverhältnis: Talgfettsäure : Diethylentriamin = 1,95 : 1.

Ausbeute: 92,5 % d.Th. Imidazolinverbindung der Formel I mit R = Ethylen ($-CH_2CH_2-$) und $R^1$ einem

Rest, der sich von der Talgfettsäure durch Abspaltung der Carboxylgruppe ableitet.

| Analyse: | gefunden | berechnet |
|---|---|---|
| N (gesamt nach Kjeldahl) | 6,9 % | 7,01 % |
| N (basisch, gesamt) | 2,22 % | 2,33 % |
| N (tertiär) | 2,15 % | 2,33 % |

Säurezahl: 2,0 (entsprechend 0,98 % freier Talgfettsäure)

Beispiel 6

In einem 2-l-Vierhalskolben mit einem Ankerrührer, Thermometer, heizbaren Tropftrichter und absteigendem Kühler werden nach dreimaligem Evakuieren und Spülen mit Stickstoff 103 g (1 mol) Diethylentriamin vorgelegt, 2,86 g 10%ige methanolische Natrium-methylat-Lösung zugegeben und unter Einleiten von Stickstoff auf eine Innnentemperatur von 115 bis 120 °C hochgeheizt. Anschließend werden aus einem heizbaren Tropftrichter 568 g (1,93 mol) Stearinsäuremethylester mit einer Temperatur von 110 °C zugegeben. Dan wird die Reaktionstemperatur im Reaktionskolben auf 150 °C gesteigert, wobei eine klare homogene Reaktionslösung entsteht und Methanol am absteigenden Kühler laufend abdestilliert wird.

Anschließend wird 6 Stunden bei 6 mbar und 188 - 190 °C kondensiert.

Das erhaltene Rohprodukt fällt mit großer Reinheit und guter Ausbeute an.

Ausbeute: 575 g entsprechend 96,6 % d.Th.,

Gehalt an 1-(2-Stearoylamino-ethyl)-2-heptadecyl-2-imidazolin: 93,13 %

| Analyse: | gefunden | berechnet |
|---|---|---|
| N (gesamt nach Kjeldahl) | 7,0 % | 7,01 % |
| N (basisch, gesamt) | 2,29 % | 2,33 % |
| N (tertiär) | 2,17 % | 2,33 % |
| N (primär) | < 0,01 % | 0 % |
| N (sekundär) | 0,12 % | 0 % |

Säurezahl: 4,7 (entsprechend 2,38 % freier technischer Stearinsäure)

Beispiel 7

In einem 5-l-VA-Druckkessel, der mit einem Thermometer, Ankerrührer und Bodenventil versehen ist, werden nach dreimaligem Evakuieren und Spülen mit Stickstoff 0,222 kg (2,15 mol) Diethylentriamin vorgelegt und auf 150 °C Innentemperatur hochgeheizt.

Dann werden aus einem heizbaren Tropftrichter 1152,9 g (4,19 mol) flüssige technische Stearinsäure (Molgewicht 275, Säurezahl 204) mit einer Temperatur von 150 °C in 90 s zugegeben und sofort das entstehende Kondensationswasser abdestilliert. Es wird keine spontane Wasserbildung und damit kein Druckanstieg beobachtet. Anschließend wird 3 h bei 160 bis 170 °C kondensiert, wobei laufend Wasser am absteigenden Kühler abdestilliert wird. Durch Erhitzen auf 180 bis 190 °C bei einem Vakuum von 0,1 mbar wird in 6 h die Reaktion beendet.

Ausbeute: 1250 g = 99,1 % d.Th.,

Gehalt an 1-(2-Stearoylamino-ethyl)-2-heptadecyl-2-imidazolin 95,7 %

Ansatzmolverhältnis: Technische Stearinsäure : Diethylentriamin = 1,9452 : 1

| Analyse: | gefunden | berechnet |
|---|---|---|
| N (gesamt nach Kjeldahl) | 6,95 % | 7,01 % |
| N (basisch, gesamt) | 2,45 % | 2,33 % |
| N (tertiär) | 2,17 % | 2,33 % |
| N (primär) | 0,28 % | 0 % |
| N (sekundär) | ≪0,01 % | 0 % |

Säurezahl: 1,2 (entsprechend 0,61 % freier technischer Stearinsäure)

Beispiel 8

In einem 3-l-Vierhalskolben mit einem Ankerrührer, Thermometer, heizbarem Tropftrichter und absteigendem Kühler werden nach dreimaligem Evakuieren und Spülen mit Stickstoff 131 g (1 mol) 1-(3-Amino-1-methyl-propyl)-ethylendiamin vorgelegt, 1,31 g unterphosphorige Säure zugefügt und auf eine Innentemperatur von 160 °C geheizt. Aus einem heizbaren Tropftrichter werden dann 533,5 g (1,94 mol) technische Stearinsäure (Säurezahl: 204) mit einer Temperatur von 150 °C in 2 min zudosiert und das entstehende Reaktionswasser laufend über Kopf am absteigenden Kühler abdestilliert. Dann wird 5 h bei 170 °C kondensiert. Bei 0,1 mbar wird durch Heizen auf 180 bis 190 °C die Cyclisierung zum Imidazolin in 8 h abgeschlossen.

Ansatzmolverhältnis: Technische Stearinsäure : 1-(3-Amino-1-methyl-propyl)-ethylendiamin = 1,94 : 1.

Ausbeute: 604 g entsprechend 98,6 % d.Th.,
Gehalt an Imidazolinverbindung der Formel I mit R = Methyl-trimethylen ($-CH(CH_3)CH_2CH_2-$) und $R^1$ = Heptadecyl: 92,7 %

| Analyse: | gefunden | berechnet |
|---|---|---|
| N (gesamt nach Kjeldahl) | 6,08 % | 6,16 % |
| N (basisch, gesamt) | 1,96 % | 2,05 % |
| N (tertiär) | 1,90 % | 2,05 % |
| N (primär) | 0,15 % | 0 % |
| N (sekundär) | ≪0,01 % | 0 % |

Säurezahl: 1,8 (entsprechend 0,88 % freier technischer Stearinsäure)

Anstelle von Stearinsäure können auch andere Fettsäuren der Formel II mit gleich gutem Erfolg eingesetzt werden.

Beispiel 9 (Vergleichsbeispiel):

In einem 5-l-VA-Druckkessel, der mit einem Thermometer, Ankerrührer und Bodenventil versehen ist, werden nach dreimaligem Evakuieren und Spülen mit Stickstoff 0,222 kg (2,15 mol) Diethylentriamin vorgelegt und 1,215 kg (4,40 mol) Stearinsäure flüssig mit einer Temperatur von 70 bis 75 °C zugegeben. Anschließend werden beide Reaktanten hochgeheizt. Dabei entsteht eine schwerrührbare Paste. Diese wird bei 135 °C klar, und bei 145 bis 150 °C setzt spontan die Reaktion ein, so daß die Reaktion nur unter einem Überdruck bis 2 bar gefahren werden kann. Nach dem Entspannen wird 6 h bei 170 bis 190 °C und 10 mbar kondensiert.

Das Ausgangsmolverhältnis beträgt: Stearinsäure : Diethylentriamin = 2,05 :1.

Ausbeute: 84,9 % d.Th. 1-(2-Stearoylamino-ethyl)-2-heptadecyl-2-imidazolin

| Analyse: | gefunden | berechnet |
|---|---|---|
| N (gesamt nach Kjeldahl) | 6,85 % | 7,01 % |
| N (basisch, gesamt) | 2,25 % | 2,33 % |
| N (tertiär) | 1,97 % | 2,33 % |
| N (primär) | 0,27 % | 0 % |
| N (sekundär) | 0,1 % | 0 % |

Säurezahl: 5 (entsprechend 2,4 % freier Stearinsäure)

Beispiel 10 (Vergleichsbeispiel):

In einem 5-l-VA-Druckkessel, der mit einem Thermometer, Ankerrührer und Bodenventil versehen ist, werden nach dreimaligem Evakuieren und Spülen mit Stickstoff 1,5 kg (5,43 mol) Stearinsäure und zusammen mit 1,66 g unterphosphoriger Säure aufgeschmolzen und auf eine Innentemperatur von 120 bis 130 °C erhitzt. Dann werden aus einem zweiten 1-l-VA-Druckkessel 295 g (2,86 mol) Diethylentriamin mit

einer Temperatur von 130° C zugedrückt. Nach 3 min setzt die Reaktion spontan unter Aufschäumen ein, und es wird am Reaktionskessel ein Druck von 1,5 bar gemessen.

Nach dem Entspannen wird bei einer Temperatur von 150 bis 170° C Wasser am absteigenden Kühler abdestilliert und bei 180 bis 190° C in 4 h die Reaktion unter 10 mbar beendet.

Ausgangsmolverhältnis: Stearinsäure : Diethylentriamin = 1,89 : 1
Ausbeute: 78,0 % d.Th. 1-(2-Stearoylamino-ethyl)-2-heptadecyl-2-imidazolin

| Analyse: | gefunden | berechnet |
|---|---|---|
| N (gesamt nach Kjeldahl) | 6,9 % | 7,01 % |
| N (basisch, gesamt) | 2,06 % | 2,33 % |
| N (tertiär) | 1,81 % | 2,33 % |
| N (primär) | 0,25 % | 0 % |
| N (sekundär) | 0,1 % | 0 % |

Säurezahl: 9,5 (entsprechend 3,44 % freier Stearinsäure)

Beispiel 11

In einem 5-l Vierhals-Rührkolben, der mit einem Ankerrührer, Thermometer, heizbarem Tropftrichter und Rückflußkühler versehen ist, werden 475 g (4,61 mol) Diethylentriamin vorgelegt und der Kolben zweimal evakuiert. Dann wird mit Stickstoff gespült und unter Rühren auf eine Innentemperatur von 150 - 155° C geheizt. Nun werden aus einem heizbaren Tropftrichter 2841 g (9,00 mol) gehärteter Rindertalg mit einer Verseifungszahl 185 mit einer Temperatur von 150 -155° C innerhalb von 5 Minuten zugegeben und anschließend 2 Stunden bei 160 - 170° C gerührt. Danach wird der Rückflußkühler durch einen absteigenden Kühler ausgetauscht und bei einer Badtemperatur von 188 - 200° C (Innentemperatur maximal 190° C) bei 2 - 3 mbar ein Gemisch aus Glycerin, Wasser und wenig Diethylentriamin bei 130 - 160° C abdestilliert.

Anschließend wird bei 188 - 190° C Innentemperatur am absteigenden Kühler 6 Stunden ständig Wasser und das restliche Glycerin abdestilliert. Das erhaltene Produkt besitzt eine hohe Reinheit und praktisch keine Nebenprodukte.

Ansatzmolverhältnis:
Rindertalg : Diethylentriamin = 1,952 : 1
Ausbeute: 94,4 % Th. Imidazolinverbindung der Formel I

| Analyse: | gefunden | berechnet |
|---|---|---|
| N (tertiär) | 2,20 % | 2,33 % |
| N (primär + sekundär) | <0,1 % | 0 |

Säurezahl: 2,9 (entsprechend 1,1 % freier Rindertalgfettsäure).

Beispiel 12:

In einem 5-l Vierhals-Rührkolben, der mit einem Ankerrührer, Thermometer, heizbarem Tropftrichter und Rückflußkühler versehen ist, werden 486,6 g (4,72 mol) Diethylentriamin vorgelegt und der Kolben zweimal evakuiert. Dann wird mit Stickstoff gespült und unter Rühren auf eine Innentemperatur von 150 - 155° C geheizt. Nun werden aus einem heizbaren Tropftrichter 2841 g (9,00 mol) gehärteter Rindertalg mit einer Verseifungszahl von 185 in 5 Minuten mit einer Temperatur von 150 - 155° C zugegeben und 2 Stunden bei 170 - 175° C gehalten. Anschließend wird der Rückflußkühler durch einen absteigenden Kühler ersetzt und bei einer Badtemperatur von 180 bis 200° C (Innentemperatur maximal 190° C) ein Gemisch aus Glyzerin, Wasser und geringe Mengen Diethylentriamin abdestilliert.

Anschließend wird 6 Stunden bei 188 - 190° C Innentemperatur am absteigenden Kühler weiter bei 2 - 3 mbar abdestilliert.

Das auf die oben beschriebene Herstellungsweise erhaltene Imidazolinprodukt zeichnet sich durch hohe Reinheit und geringe Mengen an Nebenprodukten aus.

Ansatzmolverhältnis:
Rindertalg : Diethylentriamin = 1,906 : 1.

Analyse:

$N_{tert}$: Gef.: 2,21% Ber.: 2,33 %
Imidazolingehalt: 95 %
Triamidgehalt < 4 %
Glyceringehalt: < 0,5 %
$N_{prim}$ + $N_{sec}$: < 0,1 %

Beispiel 13:

In einem 5-l Vierhals-Rührkolben, der mit einem Ankerrührer, Thermometer, heizbarem Tropftrichter und Rückflußkühler versehen ist, werden 362,3 g (3,51 mol) Diethylentriamin und 1,76 g unterphosphorige Säure vorgelegt, zweimal evakuiert, mit Stickstoff inertisiert und auf eine Innentemperatur von 140 - 141 °C gebracht.

Nun werden aus einem geheizten Tropftrichter 1295,6 g (4,69 mol) gehärtete Talgfettsäure und 635 g (0,67 mol) gehärteter Rindertalg in 60 Minuten mit einer Temperatur von 140 - 142 °C und bei einer Temperatur von 140 - 145 °C zugegeben, wobei am absteigenden Kühler laufend ein Gemisch aus Wasser und geringe Mengen Diethylentriamin abdestilliert wird. Schließlich wird noch 6 Stunden bei 2 - 3 mbar ein Gemisch aus Wasser und Glycerin bei einer Innentemperatur von 188 - 190 °C abdestilliert.

Das erhaltene Imidazolinprodukt zeichnet sich durch hohe Reinheit und geringe Konzentration an möglichen Nebenprodukten aus.

$N_{tert}$: Gef.: 2,16 % Ber.: 2,33 %
Imidazolin-Gehalt aus $N_{tert}$: 93,1 %
Tristearoyldiethylentriamin: < 4 %
$N_{prim}$ + $N_{sec}$: < 0,1 %
Glycerin: < 0,5 %
Säurezahl: 5,1 entsprechend 2,5 % freier Talgfettsäure
Umsetzungsverhältnis:

Talgfettsäure 1,908 mol : 1,0 mol Diethylentriamin, wobei sich die Talgfettsäure zusammensetzt aus
70 mol% freier Talgfettsäure und
30 mol% Talgfetttriglycerid.

Beispiel 14:

In einem 5-l Vierhals-Rührkolben, der mit einem Ankerrührer, Thermometer, heizbarem Tropftrichter und Rückflußkühler versehen ist, werden 316,7 g (3,07 mol) Diethylentriamin vorgelegt, zweimal evakuiert, mit Stickstoff inertisiert und auf eine Innentemperatur von 150 - 155 °C geheizt. Nun werden aus dem heizbaren Tropftrichter 1492,3 g (5,4 mol) Stearinsäure mit der Säurezahl 203 und einer Temperatur von 150 - 155 °C innerhalb von 120 Minuten zugefügt und laufend das während der Reaktion entstehende Wasser abdestilliert. Zusätzlich werden anschließend noch 215 g (0,22 mol) gehärteter Rindertalg ebenfalls mit einer Temperatur von 150 - 155 °C zugesetzt. Dabei destilliert ein Gemisch aus Wasser, Glycerin und geringe Mengen Diethylentriamin ab.

Die Reaktion wird weitere 6 Stunden bei 2- 3 mbar / 188 - 189 °C weitergeführt, dabei entsteht ein sehr reines Imidazolinprodukt ohne Nebenprodukte.

Gef.: $N_{tert}$ 2,12 % Ber.: $N_{tert}$ 2,33 %
Imidazolingehalt aus $N_{tert}$ berechnet: 91,0 %
Tristearoyldiethylentriamin ≮ 4 %
Glyceringehalt: < 0,5 %
$N_{prim}$ + $N_{sec}$: < 0,2 %
Säurezahl: 4,2 entsprechend 2,1 % Talgfettsäure.

Beispiel 15:

In einem 3-Liter Rührkolben, der mit einem Ankerrührer, Thermometer, heizbaren Tropftrichter und absteigenden Kühler versehen ist, werden 316,7 g (3,07 mol) Diethylentriamin vorgelegt. Es wird zweimal evakuiert und mit Stickstoff gespült, 3,15 g unterphosphorige Säure (50%ige wäßrige Lösung) zugegeben

und auf eine Innentemperatur von 155-160 °C geheizt.

Dann werden aus einem heizbaren Tropftrichter zunächst 18,9 g (0,02 mol) Talgfettsäuretriglyzerid (Verseifungszahl: 185) mit einer Temperatur von 150 °C innerhalb von 2 Minuten zugegeben und dann noch 1136,4 g (5,94 mol) gehärtete Talgfettsäure mit einer Temperatur von 155-160 °C nachgesetzt und 1 Stunde bei 153-157 °C nachgerührt.

Während der Zudosierung der Talgfettsäure destilliert laufend Wasser ab. Anschließend wird in 1 Stunde auf 180 °C geheizt und dann bei 189-190 °C in 9 Stunden bei 2 mbar die Cyclisierung zum gewünschten Imidazolin fortgeführt.

Das erhaltene Imidazolinprodukt zeichnet sich durch hohe Reinheit und eine geringe Konzentration an möglichen Nebenprodukten aus.

Ausbeute: 1960 g entsprechend 99% der Theorie

$N_{tert}$: Gef.: 2,20 % Ber.: 2,33 %

Imidazolin-Gehalt aus $N_{tert}$ berechnet: 94,4 %

Tristearoyldiethylentriamin: < 4,0 %

Glycerin: < 0,2 %

Säurezahl: 3,9 entsprechend 1,9 % gehärteter freier Talgfettsäure

Umsetzungsverhältnis:

Gehärtete Talgfettsäure : Diethylentriamin = 1,95 mol : 1,0 mol, wobei sich der Talgfettsäureanteil zusammensetzt aus

99 mol% freier gehärteter Talgfettsäure und

1 mol% freier gehärteter Talgfettsäure in Form von Talgfettsäuretriglycerid.

Beispiel 16:

In einem 3-l Rührkolben, der mit einem Ankerrührer, Thermometer, heizbaren Tropftrichter und absteigenden Kühler versehen ist, werden 316,7 g (3,07 mol) Diethylentriamin vorgelegt, zweimal evakuiert und mit Stickstoff gespült, 3,15 g unterphosphorige Säure (50%ige wäßrige Lösung) zugegeben und auf eine Innentemperatur von 155-160 °C geheizt.

Dann werden aus einem heizbaren Tropftrichter 1575,2 g (5,7 mol) gehärtete Talgfettsäure mit einer Säurezahl von 203 und einer Temperatur von 150 °C innerhalb von 70 Minuten zugegeben. Anschließend fügt man noch 94,7 g (0,1 mol) Talgfetttriglyzerid (Verseifungszahl: 185) mit einer Temperatur von 150 °C in 10 Minuten zu, wobei während der gesamten Zudosierung schon Wasser am absteigenden Kühler abdestilliert wird, dem geringe Mengen Diethylentriamin beigemischt sind.

In 1 Stunde wird auf 180 °C geheizt und anschließend 9 Stunden bei 189-190 °C kondensiert, wobei das restliche Wasser und Glycerin abdestillieren.

Ausbeute: 1660 g entsprechend 99% der Theorie

Das erhaltene Imidazolinprodukt zeichnet sich durch hohe Reinheit und geringe Konzentration an möglichen Nebenprodukten aus.

$N_{tert}$: Gef.: 2,12 % Ber.: 2,33 %

Imidazolin-Gehalt aus $N_{tert}$ berechnet: 90,9 %

$N_{prim}$ + $N_{sec}$: < 0,1 %

Tristearoyldiethylentriamin: < 4 %

Glycerin: < 0,2 %

Säurezahl: 4,0 entsprechend 2 % gehärteter freier Talgfettsäure

Umsetzungsverhältnis:

gehärtete Talgfettsäure : Diethylentriamin = 1,954 mol : 1,0 mol, wobei sich der Talgfettsäureanteil zusammensetzt aus

95 mol% freier gehärteter Talgfettsäure und

5 mol% Talgfettsäure in Form von Talgfettsäuretriglycerid.

**Patentansprüche**

1. Verfahren zur Herstellung von in 2-Stellung substituierten 1-(Acylamino-alkyl)-2-imidazolinen der allgemeinen Formel I

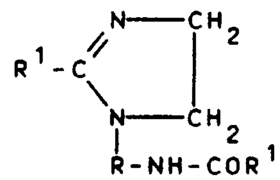

(I)

worin
R einen Alkylenrest der Formel $-CH_2-CH_2-$, $-CH_2-CH_2CH_2-$ oder $-CH(CH_3)CH_2CH_2-$ und
$R^1$ einen Fettsäurerest mit 7 bis 25 C-Atomen
bedeuten, durch Umsetzung einer Fettsäurekomponente, bestehend aus einer oder mehreren Fettsäuren der allgemeinen Formel II

$R^1COOH$ (II)

und/oder eines oder mehrerer Ester von Fettsäuren der Formel II und/oder einem oder mehreren Glyzeriden von Fettsäuren der Formel II mit einem Dialkylentriamin der allgemeinen Formel III

$H_2N-CH_2-CH_2-NH-R-NH_2$ (III)

wobei R und $R^1$ die genannten Bedeutungen besitzen, dadurch gekennzeichnet, daß die Fettsäure der allgemeinen Formel II oder ihr Ester und das Dialkylentriamin der Formel III im Molverhältnis (1,9 bis 2,0) : 1 derart umgesetzt werden, daß das Dialkylentriamin unter Intertgasatmosphäre vorgelegt und auf eine Temperatur von 100 bis 190 °C gebracht und die Fettsäurekomponente flüssig mit einer Temperatur von 100 bis 190 °C zudosiert wird und das bei der Reaktion entstehende Wasser und/oder der bei der Reaktion entstehende Alkohol und/oder Glyzerin abdestilliert und die Amidoaminbildung durch Erhitzen auf Temperaturen von 140 bis 190 °C vervollständigt und anschließend bei einem Unterdruck von mindestens 50 mbar die Cyclisierung zu der Verbindung der Formel I durchgeführt wird.

2. Verfahren nach Anspruch 1, dadurch gekennzeichnet, daß als Verbindung der Formel III Diethylentriamin der Formel XII

$H_2N-CH_2-CH_2-NH-CH_2-CH_2-NH_2$ (XII)

eingesetzt wird.

3. Verfahren nach Anspruch 1 und/oder 2, dadurch gekennzeichnet, daß bei der Zudosierung einer aus einem oder mehreren Estern von Fettsäuren der Formel II bestehenden Fettsäurekomponente das Dialkylentriamin der Formel III auf eine Temperatur von 100 bis 150 °C, bevorzugt auf 110 bis 140 °C, erhitzt und die Fettsäurekomponente mit einer Temperatur von 100 bis 150 °C, bevorzugt 110 bis 130 °C, zudosiert wird.

4. Verfahren nach Anspruch 1 und/oder 2, dadurch gekennzeichnet, daß bei der Zudosierung einer aus einer oder mehreren Fettsäuren der Formel II bestehenden Fettsäurekomponente das Dialkylentriamin der Formel III auf eine Temperatur von 130 bis 190 °C, vorzugsweise von 150 bis 170 °C, erhitzt und die Fettsäurekomponente mit einer Temperatur von 130 bis 170 °C, vorzugsweise 140 bis 160 °C, zudosiert wird.

5. Verfahren nach Anspruch 1 und/oder 2, dadurch gekennzeichnet, daß bei der Zudosierung einer aus einem oder mehreren Glyzeriden von Fettsäuren der Formel II bestehenden Fettsäurekomponente das Dialkylentriamin der Formel III auf eine Temperatur von 130 bis 190 °C, vorzugsweise von 150 bis 170 °C, erhitzt und die Fettsäurekomponente mit einer Temperatur von 130 bis 170 °C, vorzugsweise 140 bis 160 °C, zudosiert wird.

6. Verfahren nach einem oder mehreren der Ansprüche 1 bis 4, dadurch gekennzeichnet, daß das bei der Zudosierung der Fettsäurekomponente im Reaktionsgemisch entstehende Wasser und/oder der entstehende Alkohol sofort abdestilliert wird.

7. Verfahren nach einem oder mehreren der Ansprüche 1 bis 6, dadurch gekennzeichnet, daß die Cyclisierung bei einer Temperatur von 140 bis 230 °C, vorzugsweise 170 bis 210 °C, ganz besonders bevorzugt bei einer Temperatur von 180 bis 190 °C, und einem Druck von 50 bis 0,01 mbar, vorzugsweise bei einem Druck von 50 bis 0,1 mbar, durchgeführt wird.

8. Verfahren nach einem oder mehreren der Ansprüche 1 bis 7, dadurch gekennzeichnet, daß es in Anwesenheit von 0,2 bis 5 Gew.% phosphoriger und/oder unterphosphoriger Säure, bezogen auf das Gesamtgewicht der Ausgangsverbindungen der Formeln II und III, durchgeführt wird.

9. Verfahren nach einem oder mehreren der Ansprüche 1 bis 8, dadurch gekennzeichnet, daß bei der Zudosierung eines Alkylesters einer Fettsäure der Formel II 0,01 bis 2 Gew.%, vorzugsweise 0,03 bis 0,5 Gew.%, einer starken Base, bezogen auf Ester, zugesetzt wird.

**Claims**

1. Process for the preparation of 2-substituted 1-(acylaminoalkyl)-2-imidazolines of the general formula I

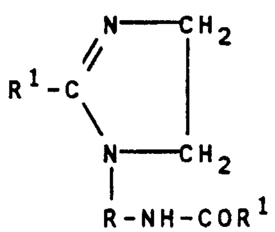

(I)

wherein
R denotes an alkylene radical of the formula $-CH_2CH_2-$, $-CH_2CH_2CH_2-$ or $-CH(CH_3)CH_2CH_2-$ and
$R^1$ denotes a fatty acid radical having 7 to 25 C atoms,
by reaction of a fatty acid component consisting of one or several fatty acids of the general formula II

$R^1COOH$ (II)

and/or one or several esters of fatty acids of formula II and/or one or several glycerides of fatty acids of formula II, with a dialkylenetriamine of the general formula III

$H_2N-CH_2-CH_2-NH-R-NH_2$ (III)

where R and $R^1$ possess the meanings mentioned, characterized in that the fatty acid of the general formula II or its ester and the dialkylenetriamine of the formula III are reacted in a molar ratio of (1.9 to 2.0) : 1 in such a way that the dialkylenetriamine is initially introduced under an inert gas atmosphere and is brought to a temperature from 100 to 190 °C and the fatty acid component is metered in as a liquid having a temperature from 100 to 190 °C and the water resulting from the reaction and/or the alcohol resulting from the reaction and/or glycerol is removed by distillation and the amidoamine formation is completed by heating to temperatures from 140 to 190 °C and the cyclization to the compound of the formula I is subsequently carried out at an under-pressure of at least 50 mbar.

2. Process according to Claim 1, characterized in that diethylenetriamine of the formula XII

$H_2N-CH_2-CH_2-NH-CH_2-CH_2-NH_2$ (XII)

is employed as the compound of the formula III.

3. Process according to Claim 1 and/or 2, characterized in that in the metering-in of a fatty acid component consisting of one or several esters of fatty acids of formula II the dialkylenetriamine of

formula III is heated to a temperature of 100 to 150 °C, preferably 110 to 140 °C, and the fatty acid component is metered in at a temperature of 100 to 150 °C, preferably 110 to 130 °C.

4. Process according to Claim 1 and/or 2, characterized in that in the metering-in of a fatty acid component consisting of one or several fatty acids of formula II the dialkylenetriamine of formula III is heated to a temperature of 130 to 190 °C, preferably 150 to 170 °C, and the fatty acid component is metered in at a temperature of 130 to 170 °C, preferably 140 to 160 °C.

5. Process according to Claim 1 and/or 2, characterized in that in the metering-in of a fatty acid component consisting of one or several glycerides of fatty acids of formula II the dialkylenetriamine of formula III is heated to a temperature of 130 to 190 °C, preferably 150 to 170 °C, and the fatty acid component is metered in at a temperature of 130 to 170 °C, preferably 140 to 160 °C.

6. Process according to one or several of Claims 1 to 4, characterized in that the water resulting from the metering-in of the fatty acid component in the reaction mixture and/or the resulting alcohol is immediately removed by distillation.

7. Process according to one or more of Claims 1 to 6, characterized in that the cyclization is carried out at a temperature of 140 to 230 °C, preferably 170 to 210 °C, very particularly preferably at a temperature from 180 to 190 °C, and at a pressure of 50 to 0.01 mbar, preferably at a pressure of 50 to 0.1 mbar.

8. Process according to one or more of Claims 1 to 7, characterized in that it is carried out in the presence of 0.2 to 5% by weight of phosphorous and/or hypophosphorous acid, relative to the total weight of the starting compounds of the formulae II and III.

9. Process according to one or more of Claims 1 to 8, characterized in that in the metering-in of an alkyl ester of a fatty acid of the formula II, 0.01 to 2% by weight, preferably 0.03 to 0.5% by weight, of a strong base, relative to ester, is added.

## Revendications

1. Procédé pour préparer des 1-(acylaminoalkyl)-2-imidazolines substituées à la position 2 qui répondent à la formule générale I :

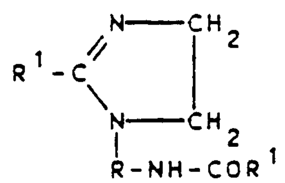

(I)

dans laquelle

R représente un radical alkylène de formule $-CH_2CH_2-$, $-CH_2CH_2CH_2-$ ou $-CH(CH_3)CH_2CH_2-$ et

$R^1$ représente un radical d'acide gras contenant de 7 à 25 atomes de carbone,

par réaction d'une composante acide gras constituée d'un ou de plusieurs acides gras de formule générale II :

$R^1COOH$ (II)

et/ou d'un ou de plusieurs esters d'acides gras de formule II et/ou d'un ou de plusieurs glycérides d'acides gras de formule II, avec une dialkylène-triamine de formule générale III :

$H_2N-CH_2-CH_2-NH-R-NH_2$ (III),

formules dans lesquelles R et $R^1$ ont les significations qui leur ont été données ci-dessus, procédé caractérisé en ce qu'on fait réagir l'acide gras de formule générale II, ou son ester, et la dialkylène-triamine de formule III dans un rapport molaire compris entre 1,9 : 1 et 2,0 : 1, et pour cela on place la dialkylène-triamine sous une atmosphère d'un gaz inerte, on la porte à une température de 100 à 190 ° C, on y ajoute progressivement la composante acide gras, à l'état liquide, à une température de 100 à 190 ° C, on chasse par distillation l'eau engendrée au cours de la réaction et/ou l'alcool engendré au cours de la réaction et/ou le glycérol, on complète la formation de l'amidoamine par chauffage à des températures de 140 à 190 ° C et on effectue ensuite la cyclisation conduisant au composé de formule I sous une dépression d'au moins 50 mbar.

2. Procédé selon la revendication 1 caractérisé en qu'on utilise, comme composé de formule III, la diéthylène-triamine, composé qui répond à la formule XII :

$H_2N-CH_2-CH_2-NH-CH_2-CH_2-NH_2$ (XII).

3. Procédé selon l'une des revendications 1 et 2 caractérisé en ce que, lorsqu'on ajoute progressivement une composante acide gras qui est constituée d'un ou de plusieurs esters d'acides gras de formule II, on chauffe la dialkylène-triamine de formule III à une température de 100 à 150 ° C, de préférence de 110 à 140 ° C, et on ajoute progressivement la composante acide gras à une température de 100 à 150 ° C, de préférence de 110 à 130 ° C.

4. Procédé selon l'une des revendications 1 et 2 caractérisé en ce que, lorsqu'on ajoute progressivement une composante acide gras constituée d'un ou de plusieurs acides gras de formule II, on chauffe la dialkylène-triamine de formule III à une température de 130 à 190 ° C, de préférence de 150 à 170 ° C, et on ajoute progressivement la composante acide gras à une température de 130 à 170 ° C, de préférence de 140 à 160 ° C.

5. Procédé selon l'une des revendications 1 et 2 caractérisé en ce que, lorsqu'on ajoute progressivement une composante acide gras constituée d'un ou de plusieurs glycérides d'acides gras de formule II, on chauffe la dialkylène-triamine de formule III à une température de 130 à 190 ° C, de préférence de 150 à 170 ° C, et on ajoute progressivement la composante acide gras à une température de 130 à 170 ° C, de préférence de 140 à 160 ° C.

6. Procédé selon une ou plusieurs des revendications 1 à 4, caractérisé en ce que l'eau formée lors de l'addition progressive de la composante acide gras au mélange réactionnel et/ou l'alcool formé sont éliminés aussitôt par distillation.

7. Procédé selon une ou plusieurs des revendications 1 à 6, caractérisé en ce que la cyclisation est effectuée à une température de 140 à 230 ° C, de préférence de 170 à 210 ° C, plus particulièrement de 180 à 190 ° C, et sous une pression de 50 à 0,01 mbar, de préférence de 50 à 0,1 mbar.

8. Procédé selon une ou plusieurs des revendications 1 à 7, caractérisé en ce qu'il est exécuté en présence de 0,2 à 5% en poids d'acide phosphoreux et/ou d'acide hypophosphoreux, par rapport au poids total des corps de départ de formules II et III.

9. Procédé selon une ou plusieurs des revendications 1 à 8, caractérisé en ce que, lorsqu'on ajoute progressivement un ester alkylique d'un acide gras de formule II, on ajoute de 0,01 à 2% en poids, de préférence de 0,03 à 0,5% en poids, d'une base forte, par rapport à l'ester.